# EUROPEAN PATENT APPLICATION

(11) **EP 3 462 168 A1**
(43) Date of publication of application: **03.04.2019**
(21) Application number: 17194104.0
(22) Date of filing: 29.09.2017
(51) Int. Cl.: G01N 27/327, G01N 33/487, H01L 29/41, B82Y 40/00

(54) **METHOD OF PRODUCING TUNNELING ELECTRODE FOR BIO-POLYMER SEQUENCING**

(71) Applicant: Siemens Healthcare GmbH, 91052 Erlangen (DE)
(72) Inventor: GUMBRECHT, Walter, 91074 Herzogenaurach (DE); PAULICKA, Peter, 91341 Röttenbach (DE)

(57) **Abstract**

The present invention relates to a method of producing tunneling electrodes using electrodeposition and forming of a short circuit, as well as tunneling electrodes produced thereby.

## Description

The present invention relates to a method of producing tunneling electrodes using electrodeposition and forming of a short circuit, as well as tunneling electrodes produced thereby.

Technologies and methods for the sequence determination of nucleic acids experienced a revolutionary development during the last 10 years. Quality improved, while costs dramatically decreased.

Nevertheless the technologies are still dominated by biological concepts such as sequencing by synthesis, making use of enzymes, e.g. polymerases, to create new nucleic acid strands, complimentary to the analyte strands (e.g. from Illumina, IonTorrent, PacBio). Several technologies are dependent on e.g. optical labels (e.g. from Illumina, PacBio). Read lengths are still too short.

Strand sequencing by feeding the target nucleic acid molecules through nanopores may overcome the read-length problem, but current technologies are still dependent on biological membranes, pore-proteins and enzymes to control the translocation-velocity of the nucleic acid-strand (e.g. from Oxford Nanopore). These biological components suffer from complex manufacturing steps, sample preparation efforts and shelf-life issues.

Already 1999, an "All Solid State and Label-Free" concept has been proposed in US 6,627,067 B1, the transverse tunneling method, which is shown as a basic concept in Fig. 1. As shown in the figure, an electrical tunneling current (Iₜᵤₙₙₑₗ) through the bio-molecule, forced by a transversal voltage Vₜ, is detected, while the biopolymer is translocated perpendicularly through the tunneling electrode junction, driven by a longitudinal voltage Vₗ.

The feasibility of discriminating the different nucleic acid (NA) bases by different tunneling currents has been shown in e.g. Ohshiro T, Matsubara K, Tsutsui M, Furuhashi M, Taniguchi M, et al. (2012) Single-molecule electrical random resequencing of DNA and RNA; Sci Rep 2: 501 and Ventra MD (2013) "Fast DNA sequencing by electrical means inches closer"; Nanotechnology 24: 342501.

Beside the controlled translocation of the bio-polymer in the longitudinal direction through the nanopore, the technical issues of manufacturing sub-nano-meter pores with aligned nanometer electrodes are not yet solved.

One interesting approach to generate nanometer sized electrode-gaps is based on the electrodeposition of metal, e.g. platinum, onto larger electrodes with larger gaps, as e.g. shown in THESE NO 5700 (2013); Towards Tunneling Electrodes for Nanopore-based DNA Sequencing, Nanogap reduction by electrodeposition; Axel Fanget; ECOLE POLYTECHNIQUE FEDERALE DE LAUSANNE; page 114.

Another approach combines electrodeposition with nanoporous structures to generate electrode-junctions and nanopores at the same time, as is e.g. presented in WO 2015010904 A1.

The electrodeposition of noble-metal onto larger electrodes has the advantage to provide very small junctions in the nm and sub-nm range, but has a severe drawback: As the method starts from larger electrodes with larger gaps, during the electrodeposition further metal is deposited, which leads to a further increase of the electrode surfaces, e.g. to several square-micrometers. Large surfaces show increased electrode capacities and increased leakage currents, though.

There remains a problem to efficiently and easily produce tunneling electrodes with high efficiency and a suitably small gap between them. The aim of the invention is particularly to find a method for the creation of nm- or sub-nm-spaced tunneling electrodes, solving the problem of large electrode-surfaces, while preferably keeping the method of electrodeposition in the environment of a semiconductor-based platform.

### Summary of the invention

The present inventors found an efficient way of producing tunneling electrodes by using a suitable combination of techniques which allow control of the distance between the tunneling electrodes at a sufficient time scale to keep the electrode surface sufficiently small enough.

The present invention relates in a first aspect to a method of producing tunneling electrodes, comprising:
- providing at least two electrodes on a substrate,
- electrodepositing a metal and/or a conductive polymer on the at least two electrodes,
- forming a short-connection between the deposited metal and/or conductive polymer on the at least two electrodes on the substrate,
- coating the deposited metal and/or conductive polymer with a self-assembly monolayer or oxidizing at least a surface layer of the deposited metal and/or conductive polymer, and
- breaking the short-connection between the deposited metal and/or conductive polymer on the at least two electrodes, preferably by electromigration.

Furthermore the present invention relates to tunneling electrodes produced by the present method.

Further aspects and embodiments of the invention are disclosed in the dependent claims and can be taken from the following description, figures and examples, without being limited thereto.

### Figures

The enclosed drawings should illustrate embodiments of the present invention and convey a further understanding thereof. In connection with the description they serve as explanation of concepts and principles of the invention. Other embodiments and many of the stated advantages can be derived in relation to the drawings. The elements of the drawings are not necessarily to scale towards each other. Identical, functionally equivalent and acting equal features and components are denoted in the figures of the drawings with the same reference numbers, unless noted otherwise.
Figure 1 shows the basic transverse tunneling concept known from the state of the art.
In Figure 2 a pair of tunneling electrodes produced by the method of the present invention is depicted schematically.
Figures 3 to 7 show schematically the production of a pair of tunneling electrodes by the method of the present invention.
Figure 8 shows in more detail a gap between two tunneling electrodes.
Figure 9 is a microscopic picture of untreated electrodes in the present Examples.
Fig. 10 is a schematic representation of the pulse process used in present Example 1.
Fig. 11 shows the electrode system in present Example 1 after the galvanization.
Fig. 12 shows I / V curves obtained in present example 2.
Fig. 13 depicts a cross-section of a typical set-up obtained in present Example 3, with protection layers present for scanning electron microscopy.

### Detailed description of the present invention

### Definitions

Unless defined otherwise, technical and scientific terms used herein have the same meaning as commonly understood by one of ordinary skill in the art to which this invention belongs.

A biopolymer is a polymer produced by a living organism, i.e. a polymeric biomolecule. The term particularly includes nucleic acid molecules.

The term "nucleic acid molecule" refers to a polynucleotide molecule having a defined sequence. It comprises DNA molecules, RNA molecules, nucleotide analog molecules and combinations and derivatives thereof, such as DNA molecules or RNA molecules with incorporated nucleotide analogs or cDNA. It also comprises cell free (cf) DNA and RNA. The term "nucleic acid sequence" relates to the sequence of nucleotides in the nucleic acid molecule. In the present description, the terms "nucleic acid molecule" and "nucleic acid" can be used interchangeably, unless clear from context that they should be differentiated.

A "translocation" of the biopolymer relates to the movement of the biopolymer within the nanoporous network layer from a location where it is captured to a different location, e.g. to a pair of tunneling electrodes. During this movement, the biopolymer can particularly be stretched so that it can be easily sequenced afterwards. The translocation of the biopolymer can be carried out by applying an electrical field that moves the biopolymer through the nanoporous network. This electrical field for translocating the biopolymer particularly is different from the electrical field for capturing the biopolymer with regard to electrical field strength and/or the direction of the electrical field, i.e. the direction of the vectors of the electrical field, at least in some locations of the electrical field, particularly a location where at least one biopolymer is present.

A "surface layer" in the present invention is the layer of the deposited metal and/or conductive polymer that comes into direct contact with the surrounding environment during the production of the tunneling electrodes, e.g. a liquid or gas.

"Electromigration" is a transport of material caused by a gradual movement of ions in a conductor due to the momentum transfer between conducting electrons and diffusing metal atoms.

In the present invention, all numerical values are considered to be complemented by the term "about".

Before the invention is described in exemplary detail, it is to be understood that this invention is not limited to the particular component parts of the process steps of the methods described herein as such methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. For example, the term "a" as used herein can be understood as one single entity or in the meaning of "one or more" entities. It is also to be understood that plural forms include singular and/or plural referents unless the context clearly dictates otherwise. It is moreover to be understood that, in case parameter ranges are given which are delimited by numeric values, the ranges are deemed to include these limitation values.

In a first aspect the present invention relates to a method of producing tunneling electrodes, comprising, particularly in this order:
- providing at least two electrodes on a substrate,
- electrodepositing a metal and/or a conductive polymer on the at least two electrodes,
- forming a short-connection between the deposited metal and/or conductive polymer on the at least two electrodes on the substrate,
- coating the deposited metal and/or conductive polymer with a self-assembly monolayer (SAM) or oxidizing at least a surface layer of the deposited metal and/or conductive polymer, and
- breaking the short-connection between the deposited metal and/or conductive polymer on the at least two electrodes, preferably by electromigration.

In this regard it is not excluded that further steps, like washing steps, are included in the present method, e.g. between different steps, e.g. after the forming of a short-connection and/or after coating with the SAM or the oxidation and/or after breaking the short, e.g. for removing excess deposition metal and/or conductive polymer, for removing excess SAM or oxidation material, and/or for removing material from breaking the short-connection, respectively.

In the present method the material of the at least two electrodes is not particularly restricted as long as it is electrically conductive and allows electrodeposition of the metal and/or conductive polymer. They can be made of a metal or metal-based material like Pt, Cu, W, Ag, AgCl, Au, Al, etc., a conductive ceramic, a conductive polymer, mixtures thereof, etc. According to certain embodiments, the at least two electrodes are made of a metal, e.g. Pt, Pd, Ag, W, Ti, Al, Au, Cu, or alloys and/or mixtures thereof.

Also the size and shape of the at least two electrodes is not particularly restricted and can be in any form or shape. The at least two electrodes can also differ in size and/or shape or be the same. For example, the at least two electrodes, e.g. two electrodes for forming a pair of tunneling electrodes, can be in the form of thin-film electrodes or top-metal electrodes, or also in the form of vias in a substrate, etc.

For example, in case of a semiconductor chip as substrate, top-metal electrodes can be placed on top of vias, which are interconnections to the topmost metal layer of the CMOS-circuitry. The electrodes may be thin-film electrodes, comprising e.g. a noble-metal such as platinum or gold with appropriate adhesion-layers to the vias, such as titanium. Also the via-metal itself, e.g. tungsten or adhesion-metals, such as titanium, could be used as electrode.

The thickness of the at least two electrodes is also not particularly restricted and can be e.g. in a range between a few nm, e.g. 10 nm, and a few µm, e.g. 10 µm. Also the size thereof can be in any range, e.g. between 10 nm and 10 µm, e.g. between 50 nm and 5 µm, e.g. from about 100 nm to about 3 µm.

The distance between the at least two electrodes is not particularly restricted as long as the material for the tunneling electrodes can be deposited thereon sufficiently, preferably within a suitable timeframe. According to certain embodiments, the distance between the at least two electrodes before the electrodeposition of the metal is between 50 nm and 5 µm, preferably between 100 nm and 3 µm. The distance can be measured as e.g. the nearest distance between any pair of electrodes of any shape, e.g. of a gap between the electrodes.

Further, also the substrate is not particularly restricted. According to certain embodiments, it is solid. According to certain embodiments, the substrate is essentially flat. According to certain embodiments, the substrate is a silicon-based or glass-based chip, e.g. also a silicon wafer or based thereon, preferably a CMOS- (complementary metal-oxide-semiconductor) chip. It can be in general a semiconductor device. The present method is especially suitable to integrate tunneling electrodes onto a silicon-chip with e.g. CMOS circuitry. This means that it is not excluded that the substrate itself comprises parts of an electric circuit, e.g. vias as discussed above, and/or even a circuit to contact electrodes provided thereon. The substrate can also comprise several layers, but also can comprise only one layer. It is further not excluded that further electrodes, detectors, etc. are comprised in the present substrate.

The metal and/or conductive polymer deposited in the present method is not particularly restricted as long as it can be sufficiently deposited and has sufficient conductivity. It is also not excluded that more than one metal and/or more than one conductive polymer are electrodeposited.

The metal can be any metal and/or an alloy thereof and/or mixtures thereof that fulfil the requirement for deposition. Also the conductive polymer can be any polymer with suitable (electrical) conductivity that fulfils the requirement for deposition, e.g. a polymer based on polypyrrole, polythiophen, polyacetylene, polyphenylene, like polyphenylene vinylene, polyphenylene sulfide, polyaniline, etc., or mixtures thereof.

According to certain embodiments the metal deposited on the at least two electrodes is a noble metal, e.g. Ag, Au, Pt and/or a mixture thereof, preferably gold.

Also the electrodeposition of the metal and/or conductive polymer is not particularly restricted. For example, the electrodeposition can be performed by alternating voltagepulses applied to a suitable plating solution which can be suitably set depending on the type of metal and/or conductive polymer. In this regard the plating solution can be suitably selected based on the metal and/or conductive polymer to be deposited. For example, for the electrodeposition of e.g. gold onto electrodes of a semiconductor chip, a suitable (e.g. commercial) gold-plating solution can be e.g. pumped through a flow-through-cell, and a suitable voltage pattern can be applied to the electrodes, which can be e.g. 20ms 800mV, 30ms open circuit, 20ms -800mV, 30ms open circuit, for formation of a electrodeposited layer. For formation of a multitude of layers, the suitable voltage pattern can be repeated and/or slightly adjusted.

In the present method it is not excluded that mixtures of metal and conductive polymer are deposited in the electrodeposition process, or that different layers of metal and conductive polymer are deposited sequentially. According to certain embodiments, the electrodeposition is carried out by depositing layer of the metal and/or conductive polymer sequentially.

According to certain embodiments, the electrodeposition is carried out in a flow-through cell. The flow-through cell in such embodiments is not particularly restricted and can be e.g. formed over the substrate with suitable dimensions for introducing a solution or suspension for electrodeposition. Also, the substrate can be mounted on a suitable carrier for the flow-through cell. The further wall materials of a flow-through cell - if one wall is the substrate, or of the carrier are not particularly restricted, and can be any suitable material, e.g. glass, plastic, etc. For example, a flow-through cell set-up can be realized by mounting a semiconductor chip onto a chip-carrier with electrical contacts and placing a flow-cell on top of the chip with fluidics inlet and outlet, e.g. connected to a pump. In this regard it is noted that also the further method steps can be performed in such a flow-through cell.

Furthermore, the forming of a short-connection, e.g. a metal bridge or a polymer bridge of the conductive polymer, between the deposited metal and/or conductive polymer on the at least two electrodes on the substrate is not particularly restricted. It can be done using a suitable voltage pattern, as e.g. described above. It can be monitored by a suitable measurement during the electrodeposition process.

According to certain embodiments, a current response to the voltage-change is monitored to indicate the formation of the short connection. Also, after the short-connection, an I/V curve measurement can be performed to characterize the short-connection, e.g. a metal-bridge or polymer-bridge, e.g. by determination of the electrical resistance. For example, a current response of the circuit in a suitable plating solution, e.g. a gold plating solution, is monitored to detect the progression of the closing gap and give a pre-indication of the short-connection. Based on these data, the voltage pattern can be adjusted, as stated above, to optimize the electrodeposition-process and create a short-connection, e.g. metal-bridge, with minimum diameter.

According to certain embodiments the electrodeposition is interrupted immediately before the short-connection. This can be done to characterize the deposited metal and/or conductive polymer, and thus the tunneling electrodes. For example, the electrodeposition process can be interrupted immediately before short-connection, as can be e.g. monitored as above, and the flow-through-cell can be flushed with a solution of [Fe(CN)₆]⁴⁻ and [Fe(CN)₆]³⁻) to characterize the electrodes.

In addition, also the coating of the deposited metal and/or conductive polymer with a self-assembly monolayer or oxidizing at least a surface layer of the deposited metal and/or conductive polymer is not particularly restricted.

According to certain embodiments, the self-assembly monolayer is generated by incubation with at least one thiol compound, particularly when gold is used as metal for electrodeposition. The at least one thiol compound is not particularly restricted and preferably is an organic thiol compound, e.g. a substituted or an unsubstituted organic thiol compound, e.g. an unsubstituted (except for the thiol group) organic C1-C20 thiol compound, preferably a linear C1-C20 thiol compound, i.e. with an alkyl residue having one to 20 C-atoms, e.g. 1-octane thiol. It can comprise also more than one thiol group, e.g. 2 or 3 thiol groups, but preferably comprises one thiol group. Also mixtures of thiol compounds can be applied, as well as only one thiol compound.

The incubation with the at least one thiol compound is not particularly restricted, and the at least one thiol compound can be e.g. applied from solution, from the gas phase, etc. A suitable solvent for a solution, e.g. of a thiol, is e.g. an alcohol like ethanol, or an amide like DMF, or mixtures thereof. For the coating of the deposited metal and/or the deposited conductive polymer the incubation with the thiol can be carried out for a suitable time, e.g. between 30 s and 30 min, e.g. between 1 and 20 min, e.g. between 2 and 15 min, e.g. between 3 and 10 min, e.g. about 5 min. After the incubation, the deposited metal and/or conductive polymer with the SAM can be washed with a suitable solvent, e.g. the one used for incubation with the thiol. For example, for coating electrodeposited gold with a self-assembly-monolayer, a solution of e.g. 1-cctanethiol in a suitable solvent, such as ethanol or DMF can be flushed into a flow-cell across the substrate, e.g. a chip, for a certain incubation time (few minutes, e.g. 5 min), and afterwards the chip can be washed with solvent.

According to certain embodiments the quality of the coating of the deposited metal with the self-assembly monolayer is determined by measuring an electrode/electrolyte capacitance of the at least two electrodes. This can be done e.g. against a further electrode. For example, the quality and performance of the SAM could be determined by measuring the electrode/electrolyte capacitance of a pair of tunneling electrodes - acting in this case as one electrode, because of the short-connection - against a 3^{rd} electrode. A dramatic decrease in electrolyte double layer capacitance would be expected as a result of a proper SAM-formation.

Alternatively, the deposited metal and/or conductive polymer can also be oxidized on at least a surface layer, e.g. of a few nm, e.g. 1 to 100 nm, e.g. 2 to 50 nm, or a monolayer, by a suitable method. For example, an oxidation can be carried out by chemical oxidation, using a suitable oxidizing agent like ozone, a peroxide, etc. Also an electrochemical oxidation is possible by supplying a suitable voltage and a suitable agent for oxidation, e.g. oxygen. In addition, also a physical oxidation is possible using e.g. a plasma, e.g. containing a source of oxygen. Again, the quality of the oxidation can be suitably determined, e.g. by measuring an electrode/electrolyte capacitance of the at least two electrodes, as above.

In the present method, an important aspect is the formation of the short-connection for forming the later tunneling junction and the further isolation of the surface of the deposited metal and/or conductive polymer afterwards. Therefore, the steps of forming a short-connection and of the coating of the deposited metal and/or conductive polymer with a SAM or oxidizing at least a surface layer thereof are preferably carried out in this order, wherein e.g. an optional washing step can be performed between these to remove material for the electrodeposition.

Furthermore, also the breaking (breakup) of the short-connection between the deposited metal and/or conductive polymer on the at least two electrodes is not particularly restricted and is preferably carried out by electromigration. In this regard a method of electromigration for the formation of nano-gaps in nano-wires is known from e.g. "Controlled fabrication of 1-2nm nanogaps by electromigration in gold and gold-palladium nanowires", F.O. Hadeed and C. Durkan, Appl.Phys.Lett. 91, 123120 (2007).

According to certain embodiments the breaking of the short-connection between the deposited metal on the at least two electrodes by electromigration is carried out by applying a suitable I/U protocol. For example, a suitable protocol can involve using a current source set to 1 mA with a limit of 10 mA and providing pulses of a suitable length, e.g. between 10 and 500 ms, e.g. between 50 and 300 ms, e.g. about 200 ms, for a suitable time until the short is sufficiently - e.g. totally - removed, which can be e.g. between 30 and 300 s, e.g. between 60 and 200 s, e.g. about 120 s.

During that procedure, due to an increasing high electrical current-density inside the short-connection, e.g. a metal-bridge, the flowing electrons are pushing away the metal-cations from their atomic lattice positions, which can create a nm-sized gap. A suitable I/U protocol can assure the desired gap, e.g. after the current spontaneously stops.

For example, the quality of the generated tunneling-junction can be determined e.g. by electrochemical measurements, such as [Fe(CN)₆]⁴⁻ / [Fe(CN)₆]³⁻) -cycling similar to before during electrodeposition. Again, a decrease in redox-currents of several orders of magnitude can be observed, which indicates that a corresponding small tunneling-current-junction has been generated.

As can be seen from the above - referring to several monitoring possibilities during electrodeposition, coating and/or breaking of the short-connection, also the formation of the tunneling electrodes in the present method can be observed by one or more suitable means which is/are not particularly restricted. According to certain embodiments, at least a current response to voltage change is monitored during the electrodeposition to indicate the formation of a short-connection.

In a further aspect the present invention is directed to tunneling electrodes that are produced by the method of the present invention. With the present method particularly at least two tunneling electrodes can be formed that have a very close proximity at at least one location between them. Further, the further surfaces thereof can be isolated with a SAM or at least an oxidized surface layer. The present, at least two, tunneling electrodes can be applied to a suitable chip and be comprised in a corresponding device for e.g. measuring biopolymers.

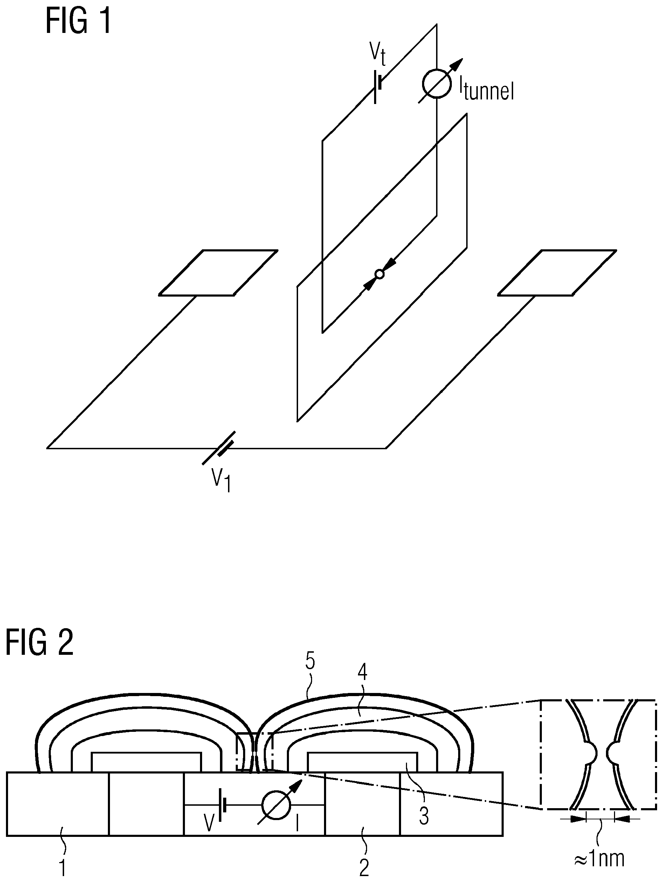

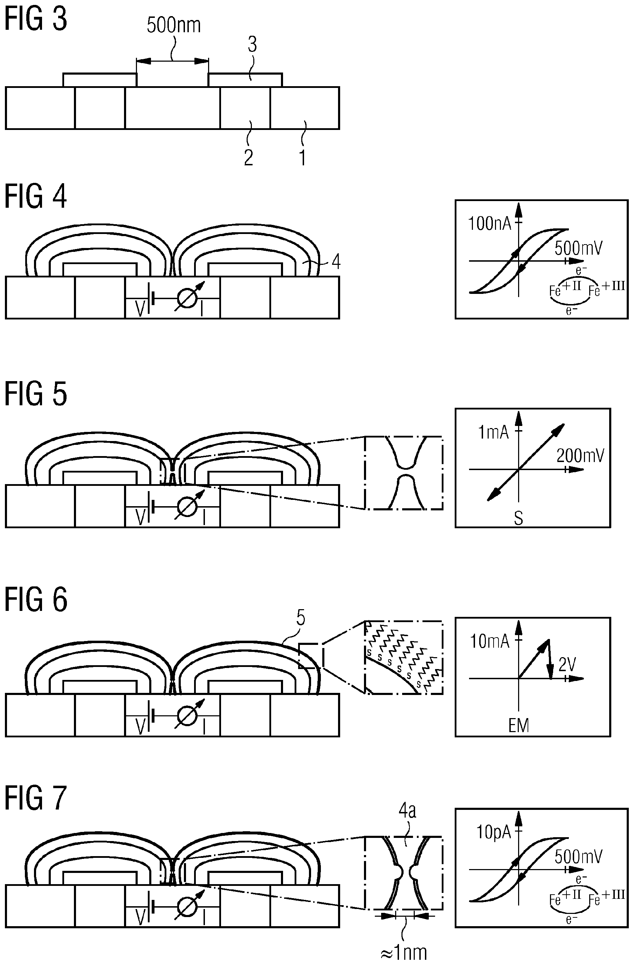

Figures 2 to 7 show an exemplary cross-section of a tunneling-electrode pair (left) and corresponding electrochemical I/V response (right in Figures 4 to 7) that is produced by the method of the present invention.

Fig. 2 shows the cross-section of the produced tunneling electrode pair, wherein the tunneling electrodes are produced on a substrate 1, e.g. a SiO₂ surface of e.g. a CMOS backend (CMOS-circuitry not shown). Through this substrate 1 a pair of vias 2 as contacts, e.g. made of a metal like W, are provided, and on top of the vias 2 top-metal electrodes 3 (one for each via), e.g. made of a metal like Pt, are provided. On top of these, or even directly on top of the vias 2, a suitable material, e.g. a metal like Au, is electrodeposited in electrodeposited layers 4, and on top thereof a SAM layer 5. As can be seen from the section view on the right, a tunneling junction is formed between the electrodeposited layers 4 at a location where a short-connection was formed beforehand and where no SAM layer 5 is present.

The pair of tunneling electrodes can be formed by steps as shown in Figures 3 to 7.

Figure 3 shows the provision of the substrate 1 with the vias 2 and the top electrodes 3 after e.g. a semiconductor thin film-processing (CMOS-circuitry not shown). The top-metal electrodes 3 of the semiconductor can be placed on top of the vias 2, which are the interconnections to the topmost metal layer of the CMOS-circuitry. The electrodes 3 may be thin-film electrodes, comprising a noble-metal such as platinum or gold with appropriate adhesion-layers to the vias, such as titanium. Also the via-metal itself, e.g. tungsten or adhesion-metals, such as titanium, could be used as electrode.

The next steps in the present method can be performed in e.g. a flow-through-cell set-up, mounting the semiconductor chip onto a chip-carrier with electrical contacts and placing a flow-cell on top of the chip with fluidics inlet and outlet, connected to a pump.

In Figure 4 the formation of electrodeposition layers 4 after electrodeposition of e.g. a noble-metal, immediately before short-connection, is shown. On the right a typical I/U-curve for electrochemical cycling of [Fe(CN)₆]⁴⁻ and [Fe(CN)₆]³⁻ during this step is schematically shown. For the electrodeposition of e.g. gold onto the electrodes of the semiconductor, a suitable (e.g. commercial) gold-plating solution can be pumped through the flow-through-cell and a suitable voltage pattern can be applied to the electrodes (e.g. 20ms 800mV, 30ms open circuit, 20ms -800mV, 30ms open circuit). The current response of the circuit in the gold plating solution can be monitored to detect the progression of the closing gap and give a pre-indication of the short-connection. Based on these data, the voltage pattern can be adjusted to optimize the electrodeposition-process and create a short connection metal-bridge with minimum diameter.

Figure 5 shows the pair of tunneling electrodes after formation of a short-connection, shown in detail in the section view in the middle. On the right the ohmic behavior with low resistance characteristic for this state is schematically shown. After short-connection, thus an I/U curve measurement can be performed to characterize the metal-bridge e.g. by determination of the electrical resistance.

Optionally, the electrodeposition process can be interrupted immediately before short-connection, and the flow-through-cell can be flushed with a solution of e.g. [Fe(CN)₆]⁴⁻ and [Fe(CN)₆]³⁻) to characterize the electrodes, as also shown in Fig. 4.

In Figure 6 the pair of tunneling electrodes after application of the self-assembly monolayer, here formed exemplary from 1-octane thiol (as shown in the section view in the middle), is depicted, with the curve on the right showing a schematic I/U-curve during a subsequent electromigration step. For the coating of the electrodeposited gold with a self-assembly-monolayer, a solution of e.g. 1-octane thiol in a suitable solvent, such as ethanol or DMF can be flushed into the flow-cell across the chip for a certain incubation time (few minutes), and afterwards the chip can be washed with solvent.

Optionally, the quality and performance of the SAM can be determined by measuring the electrode / electrolyte capacitance of both electrodes (acting as one electrode, because of the short-connection) against a 3rd electrode (not shown in Fig. 6). A dramatic decrease in electrolyte double layer capacitance would be expected as a result of a proper SAM-formation.

Figure 7 shows the finished tunneling electrode pair after electromigration, as also shown in Figure 2, with the breakup of the short-connection and the of removing thiol molecules from the junction 4a. On the right in Figure 7 again a typical I/U-curve for electrochemical cycling of [Fe(CN)₆]⁴⁻ and [Fe(CN)₆]³⁻ with currents more than 3 orders of magnitude lower compared to Figure 4 is shown. To perform the electromigration-based breakup of the short-connection junction, a suitable I/U protocol can be performed. During that procedure, due to an increasing high electrical current-density inside the metal-bridge, the flowing electrons are pushing away the metal-cations from their atomic lattice positions, which creates a nm-sized gap. The right I/U protocol can assure the desired gap, after the current spontaneously stops.

The quality of the generated tunneling-junction can be determined e.g. by electrochemical measurements, such as [Fe(CN)₆]⁴⁻ / [Fe(CN)₆]³⁻cycling, similar to Fig. 4 right. A decrease in redox-currents of several orders of magnitude, compared to 4, can be observed, which indicates that a corresponding small tunneling-current-junction has been generated.

The present tunneling electrodes can be used in a method of detecting and/or sequencing one or more biopolymers, e.g. nucleic acids like DNA. The sequencing can follow a capture and translocation process of the biopolymer in a suitable setup that comprises the present tunneling electrodes, together with e.g. suitable electrodes for capturing and/or translocating a biopolymer, as well as further support structures, e.g. a nanoporous network layer through which the biopolymer can be guided towards the tunneling electrodes, e.g. a pair of tunneling electrodes, or several pairs thereof. Also a flow channel can be provided for introducing the biopolymer. These components of the setup in which the present tunneling electrodes can be used are not particularly restricted.

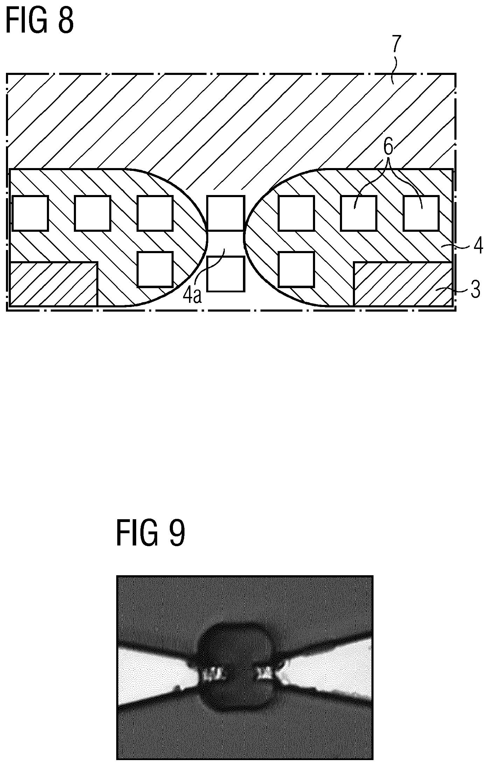

Fig. 8 shows a planar setup of tunneling electrodes with electrodeposited layers 4 on electrodes 3 and the junction 4a formed between them in such an application. For further capture and translocation of a biopolymer, a nanoporous network layer comprising nanoparticles 6, shown schematically in the figure, can be provided, which can also cover the tunneling electrodes and also assist in forming junction 4a. On top of the nanoporous network layer a fluid 7 can be provided, e.g. a hydrophobic fluid during the tunneling electrode measurement to hinder biopolymers from leaving the junction 4a. The formation and composition of such a cross-section, e.g. regarding a nanoporous network layer applied thereto, can also be taken from e.g. WO 2015010904 A. In Figure 8 especially the base electrodes 3 on which the depositied layers 4 are formed, as well as the gap 4a between the tunneling electrodes, which can also be filled with nanoparticles 6 or similar structures of the nanoporous network layer, can be seen.

In Figure 8, it is also depicted how a fluid 7 can be present on top of the tunneling electrodes 5. Such a fluid 7 can be e.g. a solvent used during introduction of a biopolymer into a setup in which the present tunneling electrodes are used, or a different solvent, e.g. a solvent like a hydrophobic liquid which can hinder a biopolymer from leaving the nanoprous network layer of particles presented by nanoparticles 6, and in particular gap 4a during detection and/or sequencing of the biopolymer. Also the junction-cross-section of the tunneling electrodes can be restricted this way.

The above embodiments can be combined arbitrarily, if appropriate. Further possible embodiments and implementations of the invention comprise also combinations of features not explicitly mentioned in the foregoing or in the following with regard to the Examples of the invention. Particularly, a person skilled in the art will also add individual aspects as improvements or additions to the respective basic form of the invention.

### Examples

The present invention will now be described in detail with reference to several examples thereof. However, these examples are illustrative and do not limit the scope of the invention.

### Example 1

In a first example, a pair of tunneling electrodes was produced in accordance with the present invention.

As an electrode system 2 electrodes with a distance of about 1 µm between them and an area of a single electrode estimated at 1.5 µm² on a CMOS-chip was used. Fig. 9 shows a microscopic picture of the untreated electrodes.

After installing the chip in a flow-through-cell the surface of the chip was cleaned with 100 mM H₂SO₄ for 2 min at a pulse voltage of 800 mV. Afterwards a washing with double distilled water was carried out.

A galvanization was carried out with a 10mM K₂[AuCl₄] solution at a pulse voltage of 800 mV and a positive pulse length of 20 ms, followed by 30 ms pause, a negative pulse length of 20 ms and a final pause of 30 ms. The total galvanization lasted about 150 s.

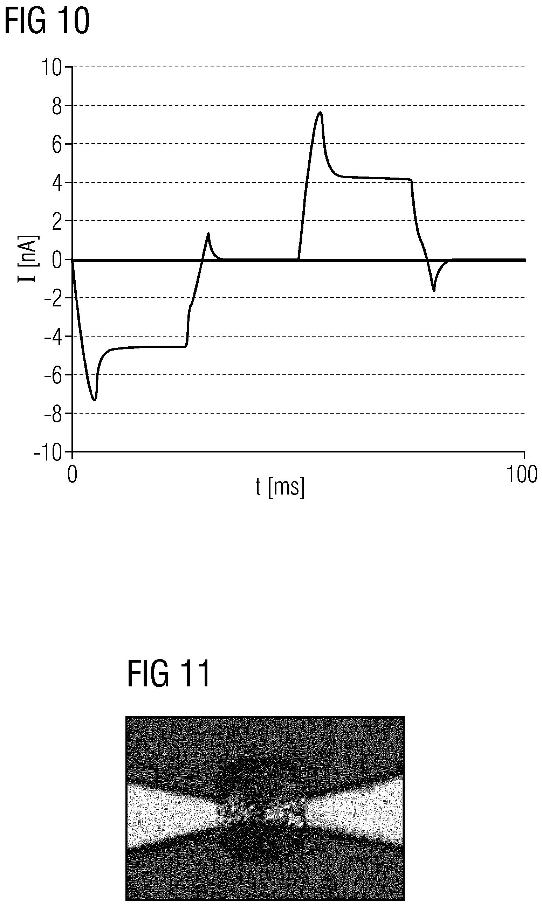

Fig. 10 shows a schematic representation of a cycle of the pulse process (x-axis: 100ms; y-axis: current I in nA) Before both electrodes grew almost together, an automatic pulse deactivation took place, and the flow cell was washed with double distilled water. Then a first cyclic voltammogram with a Fe-II Fe-III solution was recorded.

After that the flow cell was washed with double distilled water und once again flushed with a gold solution. Thereafter further galvanization pulses were applied and the automatic deactivation at an almost shortage deactivated. In this state the two electrodes could grow together, as shown in Fig. 11. Fig. 11 shows the electrode system after the galvanization.

After a further washing with double distilled water the chip was flooded with a 100 mM octane thiol solution and incubated for 5 min. Thereafter a further washing with double distilled water was carried out.

In order to "free up" ("burn") the connected position (short-connection) between the two electrodes using electro migration pulses of at most 200 ms with a current source set to 1 mA with a limit of 10 mA were applied. The process was continued till a full removal of the short location was observed after about 120 s. Then the process of the Fe-II Fe-III measurement was repeated.

Experiments have been performed, which show the corresponding decrease of redox-current according to Fig. 4 and Fig. 7.

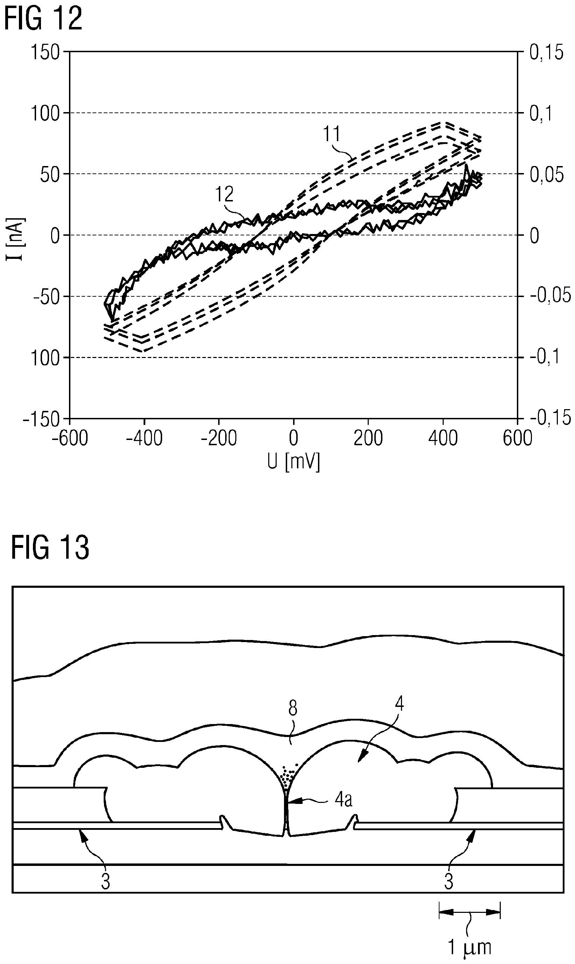

Fig. 12 shows the corresponding I / U curves, with reference number 11 (left Y-axis) referring to the measurement with the bare deposited gold surface, and reference number 12 (right Y-axis) to the one with the SAM applied. With the SAM the current was less than 1 per mill.

### Example 2

Fig. 13 shows a scanning electron micrograph of a furthermore obtained cross-section of a typical tunneling electrode setup obtained by the present method, similar to the method in Example 1 with a different shape of the tunneling electrodes, with electrodes 3, deposited layers 4, junction 4a and a protection layer 8 needed for scanning electron microscopy. The figure shows the formation of a suitable junction 4a for measuring biopolymers

The present invention provides particularly a new method of providing tunneling electrodes, using a combination of three technologies together on a CMOS-circuitry and with a controlled formation of a short-connection, offering a simple, robust and reproducible method to produce suitable tunneling junctions.

## Claims

1. A method of producing tunneling electrodes, comprising:
- providing at least two electrodes on a substrate,
- electrodepositing a metal and/or a conductive polymer on the at least two electrodes,
- forming a short-connection between the deposited metal and/or conductive polymer on the at least two electrodes on the substrate,
- coating the deposited metal and/or conductive polymer with a self-assembly monolayer or oxidizing at least a surface layer of the deposited metal and/or conductive polymer, and
- breaking the short-connection between the deposited metal and/or conductive polymer on the at least two electrodes, preferably by electromigration.

2. The method of claim 1, wherein the at least two electrodes are made of a metal, e.g. Pt, Pd, Ag, W, Ti, Al, Au, Cu, or alloys and/or mixtures thereof.

3. The method of claim 1 or 2, wherein a metal deposited on the at least two electrodes is a noble metal, preferably gold.

4. The method of any one of the preceding claims, wherein the distance between the at least two electrodes before the electrodeposition of the metal and/or conductive polymer is between 50 nm and 5 µm, preferably between 100 nm and 3 µm.

5. The method of any one of the preceding claims, wherein a current response to voltage change is monitored during the electrodeposition to indicate the formation of the short-connection.

6. The method of any one of the preceding claims, wherein the self-assembly monolayer is generated by incubation with at least one thiol compound, preferably a linear C1-C20 thiol compound.

7. The method of any one of claims 1 to 5, wherein the oxidizing of the at least surface layer of the deposited metal and/or conductive polymer is carried out by chemical, electrochemical or physical oxidation.

8. The method of any one of the preceding claims, wherein the electrodeposition is carried out in a flow-through cell.

9. The method of any one of the preceding claims, wherein the electrodeposition is interrupted immediately before the short-connection.

10. The method of any one of the preceding claims, wherein a quality of the coating of the deposited metal and/or conductive polymer with the self-assembly monolayer or oxidation of the at least a surface layer of the deposited metal and/or conductive polymer is determined by measuring an electrode/electrolyte capacitance of the at least two electrodes.

11. The method of any one of the preceding claims, wherein the breaking of the short-connection between the deposited metal and/or conductive polymer on the at least two electrodes by electromigration is carried out by applying a suitable I/U protocol.

12. Tunneling electrodes, produced by the method of any one of the preceding claims.
